# EUROPEAN PATENT APPLICATION

(11) **EP 4 324 824 A2**
(43) Date of publication of application: **21.02.2024**
(21) Application number: 23214018.6
(22) Date of filing: 05.10.2016
(51) Int. Cl.: C07D 241/24

(54) **COMBINATION ANTIBACTERIAL COMPOSITION AND SHORT-COURSE ANTIBACTERIAL REGIMEN**

(30) Priority: 14.10.2015 US 201562241280 P
(62) Divisional of application: 16855973.0
(71) Applicant: The Global Alliance for TB Drug Development, Inc., New York, NY 10005 (US)
(72) Inventor: MDLULI, Khisimuzi, Jr., Brooklyn, 11221 (US); MENDEL, Cari, M., Short Hills, 07078 (US); NUERMBERGER, Eric, Towson, 21204 (US)
(74) Representative: Vossius & Partner Patentanwälte Rechtsanwälte mbB

(57) **Abstract**

The present invention relates to therapeutic combinations of anti-bacterial agents linezolid, bedaquiline and pretomanid, and optionally with pyrazinamide, in a short-course oral dosage regimen for the treatment of tuberculosis.

## Description

### Field of the Invention

The invention relates generally to combinations of compounds with antibacterial activity and, more specifically, with anti-tuberculosis properties. In particular, it relates to chemically stable combinations of anti-bacterial agents linezolid, bedaquiline and pretomanid, and optionally with pyrazinamide, in a short-course oral dosage regimen for the treatment of tuberculosis.

All documents cited to or relied upon below are expressly incorporated herein by reference.

### Background of the Invention

*Mycobacterium tuberculosis* is the causative agent of tuberculosis ("TB"), a devastating infectious disease. It is estimated that about 2 million TB patients die each year globally. Failure to properly treat tuberculosis has caused global drug resistance in *mycobacterium tuberculosis* and thus rendering some medications ineffective.

Approximately half a million new cases of multidrug-resistant (MDR) tuberculosis occur annually (1). Current recommendations call for up to 2 years of treatment with second-line drugs that are poorly tolerated, toxic, more difficult to administer and less effective than the 6-month, so-called short course regimen for drug-susceptible TB. Regimens containing at least 6 drugs, including newer fluoroquinolones in high doses, an injectable agent, clofazimine (CFZ), pyrazinamide (PZA) and high-dose isoniazid (INH) have shown potential as effective 9-month regimens in MDR-TB cases with minimal bacillary resistance to second-line drugs (2-4). However, these regimens remain quite cumbersome to administer and are not expected to be as effective in the setting of resistance to fluoroquinolones and/or injectable agents (3).

Agents from 2 novel classes recently received conditional regulatory approval for use in MDR-TB: the diarylquinoline bedaquiline (BDQ) and the nitroimidazole derivative delamanid. Aside from some mutations known to confer cross-resistance between BDQ and CFZ (5, 6), these agents are not known to exhibit cross-resistance with other TB drugs. It was recently reported that the 3-drug regimen of BDQ plus pretomanid (PMD; formerly known as PA-824), the second nitroimidazole to enter phase 3 clinical trials, and the oxazolidinone sutezolid (SZD; formerly known as PNU-100480) have greater sterilizing activity than the first-line regimen of rifampin (RIF), INH and PZA in a murine model of TB (7-10). The 3-drug combo was more active than any of its 2-drug components, indicating that SZD contributes important activity. However, SZD has only completed a single phase 2a trial of its early bactericidal activity (8) and its further development cannot be assured.

Until recently, linezolid (LZD) was the only marketed oxazolidinone antibiotic. It has proven efficacy in salvage therapy for recalcitrant cases of MDR-TB patients, but its usage has been curtailed by dose- and duration-dependent toxicity. Novel regimens based on 3 or more oral agents, one of which is LZD, with little or no pre-existing resistance would provide simpler, more universally active regimens. Thus, a need exists in the art for novel regimens that are more effective than the current first-line regimen for drug-susceptible TB, thereby shortening and simplifying treatment for pulmonary TB irrespective of resistance to existing drugs.

### Summary of the Invention

Provided is a pharmaceutical composition, comprising a therapeutically effective amount of each of linezolid, bedaquiline and pretomanid, and optionally pyrazinamide, or a pharmaceutically acceptable salt of each thereof, and a pharmaceutically acceptable carrier.

Also provided is a method for the treatment of tuberculosis, comprising the step of administering to a patient in need thereof a therapeutically effective amount of each of linezolid, bedaquiline and pretomanid, and optionally pyrazinamide, or a pharmaceutically acceptable salt of each thereof, and a pharmaceutically acceptable carrier. In one embodiment of the invention, linezolid is removed from the treatment regimen after one to two months. In another embodiment, linezolid can be re-administered after a one to two week drug holiday.

### Detailed Description of the Invention

It is to be understood that the descriptions of the present invention have been simplified to illustrate elements that are relevant for a clear understanding of the present invention, while eliminating, for the purpose of clarity, many other elements found in typical pharmaceutical compositions. Those of ordinary skill in the art will recognize that other elements and/or steps are desirable and/or required in implementing the present invention. However, because such elements and steps are well known in the art, and because they do not facilitate a better understanding of the present invention, a discussion of such elements and steps is not provided herein. The disclosure herein is directed to all such variations and modifications to such elements and methods known to those skilled in the art. Furthermore, the embodiments identified and illustrated herein are for exemplary purposes only, and are not meant to be exclusive or limited in their description of the present invention.

Despite the conventional understanding that it is necessary to continuously administer antibacterial agents over a period of many months to obtain the desired antibacterial effect, the inventors surprising discovered that LZD in a multi-agent dosage form can be administered for one to two months and still retain sterilizing activity. More specifically, the inventors discovered that LZD adds bactericidal and sterilizing activity in formulation comprising BDQ and PMD. Its additive sterilizing activity was indistinguishable from that of SZD as shown in Example 1. In Example 3, in which the bacterial burden and the dose of PMD were higher, LZD did not appear as effective as SZD, although the difference was not statistically significant. Thus, the impact of substituting LZD for SZD in terms of duration needed to cure mice is small. This finding constitutes the basis for moving the evaluation of the BDQ+PMD+LZD regimen into patients with extensively drug-resistant TB (XDR-TB) or treatment-intolerant or non-responsive MDR-TB in the recently initiated NiX-TB trial.

Given the superior sterilizing activity of BDQ+PMD plus an oxazolidinone compared to the first-line regimen, it is also reasonable to consider whether regimens based on this combination could also shorten the treatment of drug-susceptible TB. It was previously shown that the addition of PZA to the BDQ+PMD+SZD combination produced even greater sterilizing activity, curing nearly all mice after just 6 weeks of treatment (7). However, the contribution of SZD to the 4-drug combination was not confirmed. In Example 2, it was confirmed that addition of LZD to BDQ+PZA+PMD also significantly increases the bactericidal and sterilizing activity of the regimen, curing all mice after 6 weeks of treatment, while at least 5 months of treatment with the first-line regimen is typically required to produce this result under the same conditions.

The 1.00 mg/kg dose of LZD used in the Examples produced a mean plasma AUC₀₋₂₄ₕ comparable to that produced by a dose of 600 mg twice daily in humans (19, 20), the same dose at which treatment is initiated in the NiX-TB trial. This dose has produced unacceptably high rates of myelotoxicity and neuropathy when administered for long durations in salvage regimens to treat MDR- and XDR-TB (26). Because these toxicities are dose- and duration-dependent, the inventors determined whether the contribution of LZD to the sterilizing activity of these regimens would be adversely affected by reducing the dose and duration of LZD. The potent sterilizing activity of the 4-drug regimen containing PZA in Example 2 was such that all mice were cured after 1.5 months of treatment. Nevertheless, limiting the duration of LZD 100 mg/kg to the first month did not adversely affect LZD's contribution to the sterilizing activity. In the absence of PZA, reducing the LZD dose to 50 mg/kg, which produces a mean AUC₀₋₂₄ₕ equivalent to a 600 mg daily dose in humans, reduced the bactericidal activity over the first month of treatment. However, after 2 months of treatment, only small numbers of CFU were recovered irrespective of whether LZD was administered as 100 mg/kg for 1 or 2 months or as 50 mg/kg for 2 months. Moreover, limiting LZD 100 mg/kg to the first month of treatment did not significantly increase the rate of relapse after 2 or 3 months of treatment. These results suggest that use of LZD 600 mg once daily for the first 1-2 months of treatment and/or front-loading therapy with higher doses for even shorter periods may enable LZD to contribute important bactericidal and sterilizing activity before the onset of potentially irreversible neuropathy develops (27, 28).

The inventors further discovered that linezolid can be re-administered at a lower dose after a one to two week interruption (e.g., drug or treatment holiday) based on neuropathy findings and subsequent improvement or, similarly, hematology findings. Clinical doses for pretomanid can be 200 mg qd or 100 mg qd. For bedaquiline, clinical doses can be 400 mg qd for two weeks followed by 200 mg tiw or 200 mg qd. And clinical doses for linezolid are 600 mg bid or 1200 mg qd to start.

### Certain Embodiments of the Invention

The present invention generally relates to a oral dosage combination of linezolid, bedaquiline and pretomanid, optionally with pyrazinamide.

Linezolid is a synthetic antibacterial agent of the oxazolidinone class. The chemical name for linezolid is (S)-N-[[3-[3-Fluoro-4-(4morpholinyl)phenyl]-2-oxo-5-oxazolidinyl] methyl]-acetamide. The empirical formula is C₁₆H₂₀FN₃O₄. Its molecular weight is 337.35, and its chemical structure is represented below:

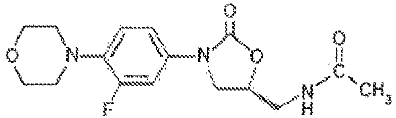

Bedaquiline is a diarylquinoline antimycobacterial drug marketed in fumarate form as SIRTURO. The chemical name of bedaquiline fumarate is (1R, 2S)-1-(6-bromo-2methoxy-3-quinolinyl)-4-(dimethylamino)-2-(1-naphthalenyl)-1-phenyl-2-butanol compound with fumaric acid (1:1). It has a molecular formula of C₃₂H₃₁BrN₂O₂•C₄H₄O₄ and a molecular weight of 671.58 (555.50 + 116.07). The molecular structure of bedaquiline fumarate is the following:

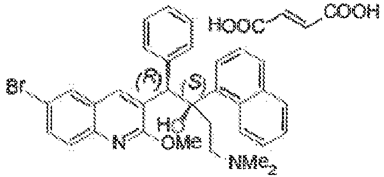

Pretomanid is a novel nitroimidazole anti-bacterial agent currently in development by Global TB Alliance. As a potential TB therapy, it has many attractive characteristics - most notably its novel mechanism of action, its activity *in vitro* against all tested drug-resistant clinical isolates, and its activity as both a potent bactericidal and a sterilizing agent. In addition, the compound shows no evidence of mutagenicity in a standard battery of genotoxicity studies, no significant cytochrome P450 interactions, and no significant activity against a broad range of Gram-positive and Gram-negative bacteria. The IUPAC designation for pretomanid is (6*S*)-2-nitro-6- {[4-(trifluoromethoxy)benzyl]oxy}-6,7-dihydro-5*H*-imidazo[2,1-*b*] [1,3]oxazine. Pretomanid has the following structure:

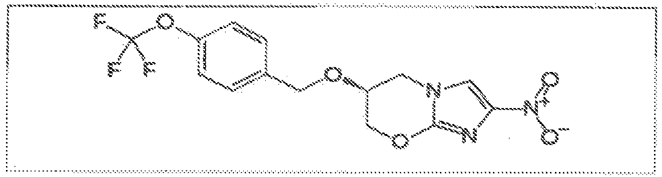

Pyrazinamide (pyrazine-2-carboxamide): 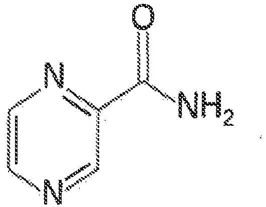 is the pyrazine analogue of nicotinamide and used as an anti-tuberculosis agent. Pyrazinamide is most commonly used for treatment of active tuberculosis (TB) during the initial phase of therapy (generally the first two months of treatment), in combination with other agents. Pyrazinamide demonstrates clinically significant antibacterial activity against *Mycobacterium tuberculosis* and *M. africanum.*

Thus, in one embodiment of the present invention, provided is a pharmaceutical composition, comprising a therapeutically effective amount of each of linezolid, bedaquiline and pretomanid, and optionally pyrazinamide, or a pharmaceutically acceptable salt of each thereof, and a pharmaceutically acceptable carrier.

In another embodiment of the invention, provided is a pharmaceutical composition wherein linezolid is at a dosage of 100 mg/kg.

In another embodiment of the invention, provided is a pharmaceutical composition wherein linezolid is at a dosage of 50 mg/kg.

In a further embodiment of the invention, provided is a method for the treatment of tuberculosis, comprising the step of administering to a patient in need thereof a therapeutically effective amount of each of linezolid, bedaquiline and pretomanid, and optionally pyrazinamide, or a pharmaceutically acceptable salt of each thereof, and a pharmaceutically acceptable carrier.

In another embodiment of the invention, provided is a method for the treatment of tuberculosis, wherein linezolid is administered for up to three months.

In another embodiment of the invention, provided is a method for the treatment of tuberculosis, wherein linezolid is administered for up to two months.

In another embodiment of the invention, provided is a method for the treatment of tuberculosis, wherein linezolid is administered for up to one month.

In another embodiment of the invention, provided is a method for the treatment of tuberculosis, wherein linezolid is administered for up to one to two months at 100 mg/kg per day.

In another embodiment of the invention, provided is a method for the treatment of tuberculosis, wherein linezolid is administered for up to two months at 50 mg/kg per day.

In another embodiment of the invention, provided is a method for the treatment of tuberculosis, wherein linezolid is administered for up to one month at 100 mg/kg per day.

In another embodiment of the invention, provided is a method for the treatment of tuberculosis, wherein linezolid is administered at a dosage of 600 mg once daily for the first one to two months.

In another embodiment,, wherein linezolid is re-administered after a one to two week drug holiday.

In a further embodiment, bedaquiline is at a dosage of 200 to 400 mg qd. In an alternative embodiment, bedaquiline is administered at 400 mg qd for two weeks followed by 200 mg tiw.

In a still further embodiment, linezolid is at a dosage of 600 mg bid or 1200 mg qd.

In another embodiment, pretomanid is at a dosage of 100 to 200 mg qd.

### Definitions and Certain Components of the Invention

When trade names are used herein, applicants intend to independently include the trade name product and the active pharmaceutical ingredient(s) of the trade name product.

The term "chemical stability" means that the three antibacterial agents in combination are substantially stable to chemical degradation. Preferably, they are sufficiently stable in physical combination to permit commercially useful shelf life of the combination product. Typically, "chemically stable" means that a first component of the mixture does not act to degrade a second component when the two are brought into physical combination to form a pharmaceutical dosage form. More typically, "chemically stable" means that the acidity of a first component does not catalyzes or otherwise accelerate the acid decomposition of a second or a third component.

The terms "synergy" and "synergistic" mean that the effect achieved with the compounds used together is greater than the sum of the effects that results from using the compounds separately, i.e. greater than what would be predicted based on the two active ingredients administered separately. A synergistic effect may be attained when the compounds are: (1) co-formulated and administered or delivered simultaneously in a combined formulation; (2) delivered by alternation or in parallel as separate formulations; or (3) by some other regimen. When delivered in alternation therapy, a synergistic effect maybe attained when the compounds are administered or delivered sequentially, e.g. in separate tablets, pills or capsules, or by different injections in separate syringes. In general, during alternation therapy, an effective dosage of each active ingredient is administered sequentially, i.e. serially, whereas in combination therapy, effective dosages of two or more active ingredients are administered together. A synergistic antibacterial effect denotes an antibacterial effect which is greater than the predicted purely additive effects of the individual compounds of the combination.

"Bioavailability" is the degree to which the pharmaceutically active agent becomes available to the target tissue after the agent's introduction into the body. Enhancement of the bioavailability of a pharmaceutically active agent can provide a more efficient and effective treatment for patients because, for a given dose, more of the pharmaceutically active agent will be available at the targeted tissue sites.

The compounds of the combinations of the invention may be referred to as "active ingredients" or "pharmaceutically active agents."

The term "prodrug" as used herein refers to any compound that when administered to a biological system generates the drug substance, i.e. active ingredient, as a result of spontaneous chemical reaction(s), enzyme catalyzed chemical reaction(s), and/or metabolic chemical reaction(s).

"Prodrug moiety" means a labile functional group which separates from the active inhibitory compound during metabolism, systemically, inside a cell, by hydrolysis, enzymatic cleavage, or by some other process (Bundgaard, Hans, "Design and Application of Prodrugs" in Textbook of Drug Design and Development (1991), P. Krogsgaard-Larsen and H. Bundgaard, Eds. Harwood Academic Publishers, pp. 113-191). Prodrug moieties can serve to enhance solubility, absorption and lipophilicity to optimize drug delivery, bioavailability and efficacy. A "prodrug" is thus a covalently modified analog of a therapeutically-active compound.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds, (1994) John Wiley & Sons, Inc., New York. Many organic compounds exist in optically active forms, i.e., they have the ability to rotate the plane of plane-polarized light. In describing an optically active compound, the prefixes D and L or R and S are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these compounds, called stereoisomers, are identical except that they are mirror images of one another. A specific stereoisomer is also referred to as an enantiomer, and a mixture of such isomers is often called an enantiomeric mixture. A 50:50 mixture of enantiomers is referred to as a racemic mixture or a racemate. The terms "racemic mixture" and "racemate" refer to an equimolar mixture of two enantiomeric species, devoid of optical activity.

The term "chiral" refers to molecules which have the property of non-superimposability of the mirror image partner, while the term "achiral" refers to molecules which are superimposable on their mirror image partner.

Any reference to any of the compounds in the compositions of the invention also includes any physiologically acceptable salt thereof. Examples of physiologically acceptable salts and their physiologically functional derivatives include salts derived from an appropriate base, such as an alkali metal (for example, sodium), an alkaline earth (for example, magnesium), ammonium and NX₄ ⁺ (wherein X is C₁-C₄ alkyl), or an organic acid such as fumaric acid, acetic acid, succinic acid. Physiologically acceptable salts of an hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound of an hydroxy group include the anion of said compound in combination with a suitable cation such as Na⁺ and NX₄ ⁺ (wherein X is independently selected from H or a C₁-C₄ alkyl group).

For therapeutic use, salts of active ingredients of the combinations of the invention will be physiologically acceptable, i.e. they will be salts derived from a physiologically acceptable acid or base. However, salts of acids or bases which are not physiologically acceptable may also find use, for example, in the preparation or purification of a physiologically acceptable compound. All salts, whether or not derived from a physiologically acceptable acid or base, are within the scope of the present invention.

The combination may be formulated in a unit dosage formulation comprising a fixed amount of each active pharmaceutical ingredient for a periodic, e.g. daily, dose or subdose of the active ingredients.

Pharmaceutical formulations according to the present invention comprise a combination according to the invention together with one or more pharmaceutically acceptable carriers or excipients and optionally other therapeutic agents. Pharmaceutical formulations containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs maybe prepared (Remington's Pharmaceutical Sciences (Mack Publishing Co., Easton, Pa.). Compositions intended for oral use may be prepared according to any method known to the art for the manufacture of pharmaceutical compositions and such compositions may contain one or more agents including antioxidants, sweetening agents, flavoring agents, coloring agents and preserving agents, in order to provide a palatable preparation. Tablets containing the active ingredient in admixture with non-toxic pharmaceutically acceptable excipient or auxiliary agents which are suitable for manufacture of tablets are acceptable. Suitable excipients or auxiliary agents include but are not limited to, for example, inert diluents, solubilizers, suspending agents, adjuvants, wetting agents, sweeteners, perfuming or flavoring substances, isotonic substances, colloidal dispersants and surfactants, including, but not limited to, charged phospholipids such as dimyristoylphosphatidylglycerin, algininic acid, alginates, acacia resin, gum arabic, 1,3-butylene glycol, benzalkonium chloride, colloidal silicon dioxide, cetosteryl alcohol, cetomacrogol emulsifying wax, casein, calcium stearate, cetylpyridine chloride, cetyl alcohol, cholesterol, calcium carbonate, CRODESTAS F-110, which is a mixture of sucrose stearate and sucrose distearate (Croda Inc.), days, kaolin and bentonite, derivates of cellulose and salts thereof, such as hydroxypropyl methylcellulose (HPMC), sodium carboxymethyl cellulose, carboxymethyl cellulose and salts thereof, methyl cellulose, hydroxyethyl cellulose, hydroxpropyl cellulose, hydroxypropyl methylcellulose phtalate, non-crystalline cellulose, dicalcium phosphate, dodecyltrimethylammonium bromide, dextrane, dialkylester of sodium sulfosuccinate (e.g. AEROSEL OT, American Cyanamid), gelatine, glycerol, glycerol monostearate, glucose, p-isononylphenoxypoly (glycidol), also known as Olin 10-G or 10-GR surfactant (Olin Chemicals, Stamford, Conn.); glucamides such as octanoyl-N-methylglucamide, decanoyl-N-methylglucamide and heptanayl-N-methylglucamide, lactose, lecithin (phosphatides), maltosides such as n-dodecyl-beta-D-maltoside, mannitol, magnesium sterarate, magnesium aluminium silicates, oils such as cotton oil, seed oil, olive oil, castor oil and sesame oil; paraffin, potato starch, polyethylene glycol (e.g. CARBOWAX 3350, CARBOWAX 1450 and CARBOPOL 9340 (Union Carbide), polyoxyethylene alkyl ester (e.g. macrogolethers such as CETOMACROGOL 1000), polyoxyethylene sorbitol fatty acid esters (e.g. TWEENS, ICI Specialty Chemicals), polyoxyethylene castor oil derivatives, polyoxyethylene stearates, polyvinyl alcohol (PVA), polyvinylpyrrolidone (PVP), phosphates, 4-(1,1,3,3-tetramethylbutyl)phenol polymer with ethylene oxide and formaldehyde (also known as TYLOXAPOL, SUPERIONE and TRITON), poloxamers and polaxamines (e.g. PLURONICS F68LF, F87, F108 and TETRONIC 908, available from BASF Corporation, Mount Olive, N.J.), pyranosides such as n-hexyl-beta-D-glucopyranoside, n-decyl-beta-D-glucopyranoside, n-octyl-beta-D-glucopyranoside, quaternary ammonium compounds, silica, sodium citrate, starches, sorbitol esters, sodium carbonate, solid polyethylene glycols, sodium dodecyl sulfate, sodium lauryl sulfate (e.g. DUPONAL P, DuPont), stearic acid, sucrose, tapioka starch, talc, thioglucosides such as n-heptyl-.beta.-D-thioglucoside, tragacanth, triethanolamine, TRITON X-200 (Rohm and Haas); and the like.

Formulations for oral use may be also presented as hard gelatin capsules where the active ingredient is mixed with an inert solid diluent, for example pregelatinized starch, calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropyl methylcelluose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxybenzoate, one or more coloring agents, one or more flavoring agents and one or more sweetening agents, such as sucrose, sucralose or saccharin.

Oil suspensions may be formulated by suspending the active ingredient in a vegetable oil, such as arachis oil, olive oil, sesame oil or coconut oil, or in a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid, BHT, etc.

Dispersible powders and granules of the invention suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

According to another embodiment of the invention, provided is a pharmaceutical composition in the form of a dispersible tablet. Dispersible tablets are intended to be dispersed in water before administration, providing a homogeneous dispersion. Dispersible tablets disintegrate within, for example, 3 minutes using water at 15-25°C.

The pharmaceutical compositions of the invention may also be in the form of oil-in-water emulsions or liposome formulations. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

The amount of active ingredient that may be combined with the carrier material to produce a single dosage form will vary depending upon the host treated and the particular mode of administration. For example, a time-release formulation intended for oral administration to humans may contain approximately 1 to 1000 mg of active material compounded with an appropriate and convenient amount of carrier material which may vary from about 5 to about 95% of the total compositions (weight:weight). The pharmaceutical, composition can be prepared to provide easily measurable amounts for administration. As noted above, formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion.

The combinations of the invention may conveniently be presented as a pharmaceutical formulation in a unitary dosage form. A convenient unitary dosage formulation contains the active ingredients in any amount from 1 mg to 1 g each, for example but not limited to, 10 mg to 500 mg for each active ingredient. In certain embodiments, 50 mg to 300 mg of each active ingredient can be used.

Segregation of active ingredients in pharmaceutical powders and granulations is a widely recognized problem that can result in inconsistent dispersions of the active ingredients in final dosage forms. Some of the main factors contributing to segregation are particle size, shape and density. Segregation is particularly troublesome when attempting to formulate a single homogenous tablet containing multiple active ingredients having different densities and different particle sizes. Glidants are substances that have traditionally been used to improve the flow characteristics of granulations and powders by reducing interparticulate friction. See Lieberman, Lachman, & Schwartz, Pharmaceutical Dosage Forms: Tablets, Volume 1, p. 177-178 (1989), incorporated herein by reference. Glidants are typically added to pharmaceutical compositions immediately prior to tablet compression to facilitate the flow of granular material into the die cavities of tablet presses. Glidants include: colloidal silicon dioxide, asbestos free talc, sodium aluminosilicate, calcium silicate, powdered cellulose, microcrystalline cellulose, corn starch, sodium benzoate, calcium carbonate, magnesium carbonate, metallic stearates, calcium stearate, magnesium stearate, zinc stearate, stearowet C, starch, starch 1500, magnesium lauryl sulfate, and magnesium oxide. The novel compositions of the present invention may contain glidants to effect and maintain homogeneity of active ingredients during handling prior to tablet compression.

Compositions of the present invention are administered to a human or other mammal in a safe and therapeutically effective amount as described herein. These safe and therapeutically effective amounts will vary according to the type and size of mammal being treated and the desired results of the treatment. A "therapeutically effective amount" is an amount effective for treating tuberculosis. The term "treating", with regard to a subject, refers to improving at least one symptom of the subject's disorder. Treating can be curing, improving, or at least partially ameliorating the disorder.

Any of the various methods known by persons skilled in the art for packaging tablets, caplets, or other solid dosage forms suitable for oral administration, that will not degrade the components of the present invention, are suitable for use in packaging. The combinations may be packaged in glass and plastic bottles. Tablets, caplets, or other solid dosage forms suitable for oral administration may be packaged and contained in various packaging materials optionally including a dessicant e.g. silica gel. Packaging may be in the form of unit dose blister packaging. For example, a package may contain one blister tray of tenofovir DF and another blister tray of emtricitabine pills, tablets, caplets, or capsule. A patient would take one dose, e.g. a pill, from one tray and one from the other. Alternatively, the package may contain a blister tray of the co-formulated combination of tenofovir DF and emtricitabine in a single pill, tablet, caplet or capsule. As in other combinations and packaging thereof, the combinations of the invention include physiological functional derivatives of tenofovir DF and FTC.

The packaging material may also have labeling and information related to the pharmaceutical composition printed thereon. Additionally, an article of manufacture may contain a brochure, report, notice, pamphlet, or leaflet containing product information. This form of pharmaceutical information is referred to in the pharmaceutical industry as a "package insert." A package insert may be attached to or included with a pharmaceutical article of manufacture. The package insert and any article of manufacture labeling provides information relating to the pharmaceutical composition. The information and labeling provides various forms of information utilized by health-care professionals and patients, describing the composition, its dosage and various other parameters required by regulatory agencies such as the United States Food and Drug Agency.

### Examples

The following examples further describe and demonstrate particular embodiments within the scope of the present invention. Techniques and formulations generally are found in Remington's Pharmaceutical Sciences (Mack Publishing Co., Easton, Pa.). The disclosure is further illustrated by the following examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

### Materials and Methods

**Mycobacterial strain.** *M. tuberculosis* H37Rv was mouse-passaged, frozen in aliquots and sub-cultured in Middlebrook 7H9 broth with 1 0% oleic acid-albumin-dextrose-catalase (OADC) (Fisher, Pittsburgh, PA) and 0.05% Tween 80 prior to infection.

**Antimicrobials.** INH, RIF, PZA, BDQ, PMD and LZD were obtained and formulated for oral administration as previously described (9, 10, 12, 13). SZD (prepared in a PEG-200/0.5% methylcellulose suspension) and TZD (prepared as phosphate prodrug dissolved in water) were synthesized by WuXi (Hubei, China). AZD (prepared as disodium phosphate prodrug dissolved in 0.3% dextrose and 0.9% saline) was provided by AstraZeneca. RWJ (prepared in 0.5% methylcellulose) was provided by Johnson and Johnson.

**Pharmacokinetics of oxazolidinones.** Uninfected female BALB/c mice weighing approximately 20 g received single doses of SZD (50 mg/kg), LZD (100 mg/kg), TZD (10 or 20 mg/kg) or AZD (50 or 200 mg/kg). Three mice per dose per time point were sampled by cardiac puncture at 0.5, 1, 2, 4, 8 and 24 hours post-dose (plus an additional 16 hour time point for AZD). Plasma drug concentrations were quantified by a validated IC/MS method performed at AstraZeneca (for AZD only) (14) or Rutgers New Jersey Medical School (for other oxazolidinones). Standards for the different oxazolidinones were provided by the sponsors. At Rutgers, analytes of interest were extracted by combining 20 µl of mouse plasma with 20 µl of acetonitrole:water (1:1), and 180 µl of methanol:acetonitrile (1:1) containing 10 ng/ml of verapamil (Sigma-Aldrich) as internal standard. The mixture was vortexed and centrifuged, and 100 µL of the supernatant was recovered and combined with 100µL of water for analysis. LC/MS-MS analysis was performed with an Agilent 1260 LC-system coupled to an AB Sciex 4000 Q-trap Mass Spectrometer (positive mode, electrospray ionization), and an Agilent column SB-C8, 2.1 x 30mm, 3.5 µm, with the column temperature fixed at 24°C. Mobile phase A was 0.1% formic acid in 100% H₂O and mobile phase B was 0.1% formic acid in 100% acetonitrile. Injection volumes were routinely 2 µl. The Mass Selective Detector was set to MRM (multiple reaction monitoring) mode using positive polarity ionization, monitoring for the ions of interest SZD (m/z 354.12/312.04), SZD-M1(370.13/238.12), LZD(338.00/235.00), and TZD(371.12/343.00) and the internal standard (m/z 455.4/165.2). The lower limit of quantification for all oxazolidinones was 5 ng/ml. PK parameters were determined by non-compartmental analysis using WinNonlin 6.4 (Certara, Princeton, NJ).

**Aerosol infection with *M. tuberculosis.*** All animal procedures were approved by the Johns Hopkins University Animal Care and Use Committee. High-dose aerosol infection was performed as previously described (15). Briefly, 5-to-6-week-old female BALB/c mice (Charles River, Wilmington, MA) were infected with *Mycobacterium tuberculosis* H37Rv, using the Inhalation Exposure System (Glas-Col, Terre Haute, IN) and a fresh log phase broth culture (optical density at 600 nm of 0.8-1.0) with the goal of implanting 3.5-4.0 log₁₀ CFU in the lungs of each mouse. Two or three mice from each aerosol infection run (Examples 1, 2, 4) or five mice from the only run (Examples 3a and 3b) were humanely killed 1 day after infection and on the day of treatment initiation (D0) to determine the number of bacteria implanted in the lungs and at the start of treatment, respectively.

**Chemotherapy.** Mice were block-randomized by aerosol run to experimental arms prior to treatment. Treatment was initiated 14-17 days after infection. Treatment was administered once daily, 5 days per week, by gavage. Except for dose-ranging monotherapy examples, drug doses (in mg/kg) were: INH (10), RIF (10), PZA (150), BDQ (25), PMD (50 or 100), SZD (50), LZD (50 or 100), TZD (10), AZD (125) and RWJ (100) (12, 14, 16-18). Each drug was administered once daily, by gavage, 5 days per week. For all combinations, PMD was administered immediately after the dose of BDQ +/- PZA, which were formulated together (9, 12). Oxazolidinones were administered at least 4 hours later.

In Example 1, control mice received RIF+INH+PZA. for 2 months followed by RIF+INH alone for a total of up to 4 months. To compare the contribution of SZD and LZD, test mice received a 3-drug combination of BDQ and PMD (50 mg/kg) plus either SZD (50 mg/kg) or LZD (100 mg/kg), or each 1- or 2-drug component comprising these 3-drug regimens, One- and two-drug regimens were limited to 1 and 2 months of treatment, respectively, except that the BDQ+PMD combination was given for up to 4 months along with the 3-drug combinations. In Example 2, the contribution of LZD to regimens containing BDQ+PMD plus PZA was assessed. Control mice received BDQ+PZA plus PMD at either 50 or 100 mg/kg. Test mice received BDQ+PZA+PMD (100 mg/kg) plus LZD (100 mg/kg) for up to 2 months. One cohort received LZD for the entire 2 months. Another received LZD for the first month only.

**Assessment of treatment efficacy.** Efficacy was assessed on the basis of lung CFU counts at selected time points during treatment (a measure of bactericidal activity) and the proportion of mice with culture-positive relapse after treatment completion (a measure of sterilizing activity). Quantitative cultures of lung homogenates were performed in parallel on 7H11 agar enriched with OADC (basic agar) and on basic agar supplemented with 0.4% activated charcoal to reduce drug carryover effects (12). Plates were incubated for up to 42 days at 37°C before final CFU counts were determined. Lung CFU counts were assessed in four or five mice per treatment group at each time point in Examples 1 and 2, respectively. The proportion of mice with culturepositive relapse was determined by holding cohorts of 15-20 mice for 3 additional months after completion of treatment, then sacrificing them to determine the proportion with positive lung cultures, as defined by ≥1 CFU of *M. tuberculosis* detected after plating the entire lung homogenate onto five 7H11 plates with and without 0.4% activated charcoal.

**Statistical analysis.** CFU counts (x) were log-transformed as (*x*+1) before analysis and group means were compared by one-way analysis of variance with Dunnett's post-test to control for multiple comparisons. Group relapse proportions were compared using Fisher's exact test, adjusting for multiple comparisons. GraphPad Prism version 5 (GraphPad, San Diego, CA) was used for all analyses. Use of 15 mice per group for relapse assessment provides greater than 80% power to detect 40 percentage point differences in the relapse rate, after setting a at 0.01 to adjust for up to 5 simultaneous two-sided comparisons. Smaller differences may not be meaningful in terms of shortening the duration of treatment.

### Example 1

### Contribution of LZD and SZD to novel combinations with BDQ+PMD

Example 1 was performed to evaluate whether the marketed oxazolidinone LZD is able to replace SZD in this combination without loss of efficacy and to clarify the contribution of each drug component to the activity of the combination.

*Lung CFU counts during treatment.* The mean CFU count (± S.D.) at the start of treatment was 6.17 ± 0.27. The lung CFU counts after 1, 2, and 3 months of treatment are presented in Table 1:

**Table 1**

| **Lung CFU counts assessed during treatment and proportion of mice relapsing after treatment completion** | | | | | | | | |
|---|---|---|---|---|---|---|---|---|
| | **Mean (±SD) log₁₀ CFU count at^{a}:** | | | | | **Proportion (%) relapsing after treatment for:** | | |
| **Drug Regimen** | **D-13** | **D0** | **M1** | **M2** | **M3** | **2 mos** | **3 mos** | **4 mos** |
| Untreated | 2.69±0.13 | 6.17±0.27 | 6.47±0.06 | | | | | |
| 2R1F+INH+PZA/ RIF+INH | | | 3.47±0.37 | 1.59±0.25 | 0.50±0.51 | | 13/15 (87) | 1/20 (5) |
| BDQ | | | 3.24±0.25 | | | | | |
| PMD | | | 4.57±0.22 | | | | | |
| LZD | | | 4.97±0.26 | | | | | |
| SZD | | | 3.85±0.37 | | | | | |
| BDQ+PMD | | | 4.21±0.40 | 1.62±0.19 | 4.520±.36 | 15/15 (100) | 10/15 (60) | 2/20 (10) |
| BDQ+LZD | | | 2.82±0.15 | 1.91±0.66 | | | | |
| BDQ+SZD | | | 2.88±0.07 | 0.65±0.50 | | | | |
| PMD+LZD | | | 3.23±0.41 | 1.48±0.12 | | | | |
| PMD+SZD | | | 1.65±0.33 | 0.23±0.40 | | | | |
| BDQ+PMD+LZD | | | 3.28±0.65 | 0.34±0.41 | 0.00±0.00 | 12/15 (80) | 0/14 (0) | 0/20 (0) |
| BDQ+PMD+SZD | | | 0.94±0.14 | 0.00±0.00 | | 14/20 (70) | 1/14 (7) | |

| | | | | | | | | |
|---|---|---|---|---|---|---|---|---|
| ^{a} Time points are shown in days (e.g., D-13, day-13; D0, day 0) or months (e.g., M1, 1 month) of treatment. | | | | | | | | |

As expected, RIF+INH+PZA reduced the mean lung CFU count by 2.70 log₁₀ and by 4.58 log₁₀ after 1 and 2 months of treatment, respectively, and left fewer than 10 CFU/mouse after 3 months of treatment. Due to the previously described antagonism of PMD on BDQ activity (7, 12), BDQ+PMD had lower activity than BDQ alone over the first month. However, its activity was virtually identical to that of the first-line regimen over 2-3 months. Addition of SZD significantly increased the initial bactericidal activity of BDQ+PMD (p< 0.001), rendering all mice culture-negative between 1 and 2 months of treatment. The activity of BDQ+PMD+SZD was significantly greater than that of RIF+INH+PZA after 1 and 2 months of treatment (p< 0.001). Addition of LZD also significantly increased the activity of BDQ+PMD (p< 0.01) and produced activity superior to that of RIF +INH+PZA after 2 and 3 months of treatment (p< 0.001). All 2-drug combinations had inferior activity compared to the 3-drug combinations of BDQ+PMD plus SZD or LZD at 2 months (p< 0.01), confirming that each component drug contributes to the efficacy of the 3-drug combinations. However, the 3-drug LZD-containing regimen was only superior to BDQ+I.ZD after 2 months of treatment. LZD-containing regimens produced higher CFU counts compared to their SZD-containing comparator regimen.

*Relapse after treatment completion.* The relapse results are displayed in Table 1. Treatment with the first-line regimen for 3 and 4 months resulted in relapse of 13 (87%) of 15 and 1 (5%) of 20 mice, respectively. The higher cure rate compared to previous examples is due to the lower than usual bacterial burden at the initiation of treatment. Consistent with the CFU count results, treatment with BDQ+PMD produced relapse results similar to the first-line regimen after 3 and 4 months but did not prevent relapse after just 2 months of treatment. However, unlike the more rapid decline in CFU counts with the 3-drug regimen containing SZD compared to LZD, the two BDQ+PMD+oxazolidinane regimens had similar sterilizing activity. While only the difference between the SZD-containing regimen and BDQ+PMD alone was statistically significant after 2 months of treatment, both oxazolidinone-containing regimens resulted in significantly fewer relapses after 3 months of treatment compared to both BDQ+PMD and the first-line regimen.

### Example 2

### Contribution of LZD to BDQ+PZA+PMD

It was previously reported the potent sterilizing activity of HDQ+PZA+SZD in mice and the additive sterilizing activity of PMD (7, 9, 10, 12). After observing that LZD had comparable sterilizing activity to SZD in combination with BDQ+PMD in Example 1, LZD could also replace SZD in the BDQ+PZA+PMD+SZD combination. The mean CFU count at the start of treatment was 7.92 ± 0.26. The lung CFU count and relapse results are displayed in Table 2:

**Table 2**

| **Lung CFU counts assessed during treatment and proportion of mice relapsing after treatment completion** | | | | | | |
|---|---|---|---|---|---|---|
| | **Mean (±SD) log₁₀ CFU count at^{a}:** | | | | **Proportion (%) relapsing after treatment for:** | |
| **Regimen** | **D-13** | **D0** | **M1** | **M2** | **1.5 mos** | **2 mos** |
| Untreated | 4.42 ±0.15 | 7.92+0.2 6 | | | | |
| RIF+INH+PZA | | | | 2.06±0.3 7 | | |
| BDQ+PZA+PMD₅₀ | | | 2.91±0.3 3 | 0.95±0.3 8 | | |
| BDQ+PZA+PMD | | | 2.93±0.3 1 | 0.06±0.1 3 | 9/15 (60) | 1/15 (7) |
| 1 BDQ+PZA+PMD+LZ D/ 1 BDQ+PZA+PMD | | | 0.11±0.2 4 | | 0/15 (0) | 0/15 (0) |
| BDQ+PZA+PMD+LZ D | | | | | 0/15 (0) | 1/15 (7) |

| | | | | | | |
|---|---|---|---|---|---|---|
| ^{a} Time points are shown in days (e.g., D-13, day-13; D0, day 0) or months (e.g., M1, 1 month) of treatment. | | | | | | |

As previously observed, BDQ+PZA+PMD had significantly greater bactericidal activity compared to RIF+INH+PZA (p< 0.001). Greater activity was observed when PMD was dosed at 100 rather than 50 mg/kg, but the difference was only noted after 2 months of treatment (p< 0.01). The higher PMD dose helped render all mice but one culture negative at this time point. The addition of LZD had a dramatic effect on efficacy, significantly reducing the CFU count at 1 month (p< 0.001) and rendering all mice but one culture-negative one month earlier. The addition of LZD to BDQ+PZA+PMD also significantly reduced the proportion of mice relapsing after 1.5 months of treatment from 60% to 0% (p< 0.001), irrespective of whether LZD was discontinued after the first month of treatment.

### Example 3

### Contribution of oxazolidinones to novel combinations with BDQ+PMD

Example 3 was performed to confirm the additive sterilizing activity of SZD and LZD when added to BDQ+PMD in Example 1 and to evaluate whether reducing the LZD dose by 50% or limiting the duration of LZD to the first 1-2 months would significantly affect its contribution.

*Lung CFU counts during treatment.* The mean CFU count at the start of treatment was 7.74 ± 0.20. The lung CFU counts observed after 1, 2 and 3 months of treatment are presented in Table 3. RIF+INH+PZA reduced the mean lung CFU count by almost 6 log₁₀ over 2 months of treatment. BDQ+PMD alone had modestly lower activity over this period. As previously observed, addition of SZD 50 mg/kg conferred superior activity compared to LZD 100 mg/kg, although both oxazolidinones significantly increased the initial bactericidal activity of BDQ+PMD. However, results with BDQ+PMD+LZD 100 mg/kg were similar whether LZD was discontinued after 1 month or continued for 2 months. Similarly, use of LZD 50 mg/kg in place of 100 mg/kg resulted in higher CFU counts at 1 month, but not 2 months, suggesting that the total LZD dose achieved with 100 mg/kg for 1 month or 50 mg/kg for 2 months sufficiently maximized the contribution of LZD to the regimen. RWJ at 100 mg/kg performed very similarly to LZD at 50 mg/kg, whereas AZD and TZD were somewhat less effective. Compared to BDQ+PMD alone, all oxazolidinones significantly increased bactericidal activity at 1 and 2 months, except TZD at the 1-month time point (p< 0.05 for AZD and for TZD at M2; p< 0.001 for other oxazolidinones).

*Relapse after treatment completion.* The relapse results are displayed in Table 3:

**Table 3**

| **Lung CFU counts assessed during treatment and proportion of mice relapsing af treatment completion ter** | | | | | | |
|---|---|---|---|---|---|---|
| | **Mean (±SD) log₁₀ CFU count at^{a}:** | | | | **Proportion (%) relapsing after treatment for:** | |
| **Regimen** | **D-13** | **D0** | **M1** | **M2** | **2 mos** | **3 mos** |
| Untreated | 3.96±0.0 8 | 7.74±0.2 0 | | | | |
| 2RIF+INH+PZA/ 1RIF+INH | | | | 1.94±0.2 7 | | 8/14 (57) |
| BDQ+PMD | | | 4.48±0.2 0 | 2.33±0.3 0 | | 3/14 (21) |
| BDQ+PMD+TZD | | | 4.20±0.1 3 | 1.67±0.4 1 | | |
| BDQ+PMD+AZD | | | 4.07±0.3 6 | 1.43±0.3 6 | | |
| BDQ+PMD+RWJ | | | 3.63±0.1 8 | 0.54±0.4 1 | | |
| BDQ+PMD+LZD ₅₀ | | | 3.48±0.3 6 | 0.39±0.2 6 | | |
| 1BDQ+PMD+LZ D₁₀₀/ BDQ+PMD | | | 2.69±0.3 7 | 0.93±0.4 9 | 9/15 (60) | 0/15 (0) |
| 2BDQ+PMD+LZ D₁₀₀/ BDQ+PMD | | | | 0.66±0.3 9 | 6/15 (40) | 0/15 (0) |
| 2BDQ+PMD+LZ D₁₀₀/ BDQ+PMD+LZD ₅₀ | | | | | | 0/12 (0) |
| BDQ+PMD+LZD ₁₀₀ | | | | | | 0/15 (0) |
| BDQ+PMD+SZD | | | 1.88±0.2 2 | 0.00±0.0 0 | 1/14 (7) | 0/14 (0) |
| Time points are shown in days (e.g., D-13, day-13; D0, day 0) or months (e.g., M1, 1 month) of treatment. | | | | | | |

Treatment with the first-line regimen for 3 months resulted in relapse of 8 (57%) of 14 mice. Treatment with BDQ+PMD produced numerically superior results, with just 3 (21%) of 14 mice relapsing at 3 months, although this difference was not statistically significant. Addition of SZD to BDQ+PMD resulted in only one relapse after just 2 months of treatment, a result that was at least as effective as 3 months of BDQ+PMD and more effective than 3 months of the first-line regimen. Addition of SZD at 50 mg/kg also resulted in fewer relapses than did the addition of LZD 100 mg/kg for 2 months, although the difference did not reach statistical significance. Use of LZD at 100 mg/kg for the first month only was less effective than SZD for 2 months, but not significantly different from LZD 100 mg/kg for 2 months. Similarly, among regimens administered for 3 months, no relapse was observed irrespective of whether LZD 100 mg/kg was continued throughout or discontinued after 1 or 2 months, or replaced with LZD 50 mg/kg after 2 months. In a *post hoc* analysis comparing BDQ+PMD to all BDQ+PMD+LZD regimens, the addition of LZD 100 mg/kg for at least one month resulted in fewer relapses after 3 months of treatment.

### Summary of Examples 1-3:

Oxazolidinone pharmacokinetics in BALB/c mice are presented in Table 4:

**Table 4**

| **Pharmacokinetic parameters after single dose oral administration of autezolid, linezolid, tedizolid or AZD5847 in BALB/c mice** | | | | | |
|---|---|---|---|---|---|
| **Compound (dose, mg/kg)** | | **t1/2 hr** | **Tmax hr** | **Cmax ng/mL** | **AUC0-24hr hr*ng/ml** |
| **Sutezolid** | **Mean** | **2.8** | **0.5** | **1292** | **3359** |
| **50** | SD | 0.5 | 0.0 | 707 | 499 |
| **Sutezolid M1** | **Mean** | | **0.5** | **15233** | **31945** |
| | SD | | 0.0 | 7875 | 5853 |
| **Linezolid** | **Mean** | **3.0** | **0.5** | **81933** | **243547** |
| **100** | **SD** | 2.5 | 0.0 | 32240 | 31577 |
| **Tedizolid** | **Mean** | **3.0** | 0.7 | **6417** | **40414** |
| **10** | SD | 0.4 | 0.3 | 624 | 2942 |
| **Tedizolid** | **Mean** | **3.9** | **0.5** | **11433** | **77698** |
| **20** | SD | 1.4 | 0.0 | **1665** | 6107 |
| **AZD5847** | **Mean** | **2.4** | **0.7** | **20433** | **74761** |
| **50** | SD | 0.2 | 0.3 | 2079 | 6138 |
| **AZD5847** | **Mean** | **8.6** | **0.7** | **31367** | **220246** |
| **200** | SD | 2.9 | 0.3 | 1270 | 34953 |

As previously described (18), oral administration of SZD at 50 mg/kg resulted in rapid and extensive metabolism to the active sulfoxide M1 metabolite (also known as PNU-101603) for which the mean AUC₀₋₂₄ₕ and Cₘₐₓ were approximately 10 times higher than that of the SZD parent. Overall, the SZD and SZD M1 AUC₀₋₂₄ₕ values were similar to or, in the case of SZD, modestly lower than the geometric means observed in TB patients receiving 1,200 mg per day (8). LZD showed the highest exposure among the tested compounds with AUC₀₋₂₄ₕ of 244 hr*ug/mL that is comparable to the average among TB patients receiving 1,200 mg daily (19, 20). TZD exposure was dose proportional from 10 mg/kg to 20 mg/kg. For the 10 mg/kg dose evaluated in the efficacy study, the total drug TZD AUC₀₋₂₄ₕ of 40 hr*ug/mL was similar to that determined in other infection models (21-23). Interpolating between the mean AZD AUC₀₋₂₄ₕ values produced by the 50 and 200 mg/kg doses, the predicted AUC₀₋₂₄ₕ of 160-190 hr*ug/mL produced by the 125 mg/kg dose used in the combination efficacy study (Example 3 below) is similar to the steady state AUC₀₋₂₄ₕ in healthy human volunteers receiving 800 mg twice daily (24), the highest dose administered in a recent dose-ranging trial of early bactericidal activity. The oxazolidinones were cleared relatively rapidly, with t_{1/2} values between 2.8 to 3.9 hrs. AUC₀. ₂₄ₕ values from all the compounds were approximately equal to AUC_{inf} values (≥ 98%), suggesting that no accumulation would be expected after multiple doses in BALB/c mice.

The invention is further described in the following numbered paragraphs:
1. A pharmaceutical composition, comprising a therapeutically effective amount of each of linezolid, bedaquiline and pretomanid, and optionally pyrazinamide, or a pharmaceutically acceptable salt of each thereof, and a pharmaceutically acceptable carrier. [ADD CLINICAL DOSAGES].
2. The pharmaceutical composition according to paragraph 1, wherein linezolid is at a dosage of 100 mg/kg.
3. The pharmaceutical composition according to paragraph 1, wherein linezolid is at a dosage of 50 mg/kg.
4. A method for the treatment of tuberculosis, comprising the step of administering to a patient in need thereof a therapeutically effective amount of each of linezolid, bedaquiline and pretomanid, and optionally pyrazinamide, or a pharmaceutically acceptable salt of each thereof, and a pharmaceutically acceptable carrier.
5. The method according to paragraph 4, wherein linezolid is administered for up to three months.
6. The method according to paragraph 4, wherein linezolid is administered for up to two months.
7. The method according to paragraph 4, wherein linezolid is administered for up to one month.
8. The method according to paragraph 4, wherein linezolid is administered for up to one to two months at 100 mg/kg per day.
9. The method according to paragraph 4, wherein linezolid is administered for up to two months at 50 mg/kg per day.
10. The method according to paragraph 4, wherein linezolid is administered for up to one month at 100 mg/kg per day.
11. The method according to paragraph 4, wherein linezolid is administered at a dosage of 600 mg once daily for the first one to two months.
12. The method according to paragraph 4, wherein linezolid is re-administered after a one to two week drug holiday.
13. The pharmaceutical composition according to paragraph 1, wherein bedaquiline is at a dosage of 200 to 400 mg qd.
14. The method according to paragraph 4, wherein bedaquiline is administered at 400 mg qd for two weeks followed by 200 mg tiw.
15. The pharmaceutical composition according to paragraph 1, wherein linezolid is at a dosage of 600 mg bid or 1200 mg qd.
16. The pharmaceutical composition according to paragraph 1, wherein pretomanid is at a dosage of 100 to 200 mg qd.

### References

1. World Health Organization. 2014. Multidrug-resistant tuberculosis (MDR-TB) 2014 Update. Geneva, Switzerland.
2. Aung KJ, Van Deun A, Declercq E, Sarker MR, Das PK, Hossain MA, Rieder HL. 2014. Successful '9-month Bangladesh regimen' for multidrug-resistant tuberculosis among over 500 consecutive patients. Int J Tuberc Lung Dis 18:1180-1187.
3. Nuermberger E, Yew WW. 2015. Expanding the evidence base supporting shorter treatment durations for multidrug-resistant tuberculosis. Int J Tuberc Lung Dis 19:497-498.
4. Van Deun A, Maug AK, Salim MA, Das PK, Sarker MR, Daru P, Rieder HL. 2010. Short, highly effective, and inexpensive standardized treatment of multidrug-resistant tuberculosis. Am J Respir Crit Care Med 182:684-692.
5. Andries K, Villellas C, Coeck N, Thys K, Gevers T, Vranckx L, Lounis N, de Jong BC, Koul A. 2014. Acquired resistance of Mycobacterium tuberculosis to bedaquiline. PLoS One 9:el02135.
6. Hartkoorn RC, Uplekar S, Cole ST. 2014. Cross-Resistance between Clofazimine and Bedaquiline through Upregulation of MmpL5 in Mycobacterium tuberculosis. Antimicrobial Agents and Chemotherapy 58:2979-2981.
7. Tasneen R, Williams K, Amoabeng 4, Minkowski A, Mdluli KE, Upton AM, Nuermberger EL. 2015. Contribution of the Nitroimidazoles PA-824 and TBA-354 to the Activity of Novel Regimens in Murine Models of Tuberculosis. Antimicrobial Agents and Chemotherapy 59:129-135.
8. Wallis RS, Dawson R, Friedrich SO, Venter A, Paige D, Zhu T, Silvia A, Gobey J, Ellery C, Zhang Y, Eisenach K, Miller P, Diacon AH. 2014. Mycobactericidal Activity of Sutezolid (PNU-100480) in Sputum (EBA) and Blood (WBA) of Patients with Pulmonary Tuberculosis. PLoS ONE 9:e94462.
9. Williams K, Minkowski A, Amoabeng O, Peloquin CA, Taylor D, Andries K, Wallis RS, Mdluli KE, Nuermberger EL. 2012. Sterilizing Activities of Novel Combinations Lacking First- and Second-Line Drugs in a Murine Model of Tuberculosis. Antimicrobial Agents and Chemotherapy 56:3114-3120.
10. Williams KN, Brickner SJ, Stover CK, Zhu T, Ogden A, Tasneen R, Tyagi S, Grosset JH, Nuermberger EL. 2009. Addition of PNU-100480 to First-Line Drugs Shortens the Time Needed to Cure Murine Tuberculosis. American Journal of Respiratory and Critical Care Medicine 180:371-376.
11. Hilliard JJ, Fernandez J, Melton J, Macielag MJ, Goldschmidt R, Bush K, Abbanat D. 2009. In vivo activity of the pyrrolopyrazolyl-substituted oxazolidinone RWJ-41 6457. Antimicrob Agents Chemother 53:2028-2033.
12. Tasneen R, Li S-Y, Peloquin CA, Taylor D, Williams KN, Andries K, Mdluli KE, Nuermberger EL. 2011. Sterilizing Activity of Novel TMC207- and PA-824-Containing Regimens in a Murine Model of Tuberculosis. Antimicrobial Agents and Chemotherapy 55:5485-5492.
13. Tyagi S, Nuermberger E, Yoshimatsu T, Williams K, Rosenthal I, Lounis N, Bishai W, Grosset J. 2005. Bactericidal activity of the nitroimidazopyran PA-824 in a murine model of tuberculosis. Antimicrob Agents Chemother 49:2289-2293.
14. Balasubramanian Y, Solapure S, Shandil It, Gaonkar S, Mahesh KN, Reddy J, Deshpande A, Bharath S, Kumar N, Wright L, Melnick D, Butler SL. 2014. Pharmacokinetic and pharmacodynamic evaluation of AZD5847 in a mouse model of tuberculosis. Antimicrob Agents Chemother 58:4185-4190.
15. Nuermberger E, Tyagi S, Tasneen R, Williams KN, Almeida D, Rosenthal I, Grosset JH. 2008. Powerful bactericidal and sterilizing activity of a regimen containing PA-824, moxifloxacin, and pyrazinamide in a murine model of tuberculosis. Antimicrob Agents Chemother 52:1522-1524.
16. Drusano GL, Liu W, Kulawy R, Louie A. 2011. Impact of granulocytes on the antimicrobial effect of tedizolid in a mouse thigh infection model. Antimicrob Agents Chemother 55:5300-5305.
17. Rouan M-C, Lounis N, Gevers T, Dillen L, Gilissen R, Raoof A, Andries K. 2012. Pharmacokinetics and Phannacodynamics of TMC207 and Its N-Desmethyl Metabolite in a Murine Model of Tuberculosis. Antimicrobial Agents and Chemotherapy 56:1444-1451.
18. Williams KN, Stover CK, Zhu T, Tasneen R, Tyagi S, Grosset JH, Nuermberger E. 2009. Promising Antituberculosis Activity of the Oxazolidinone PNU-100480 Relative to That of Linezolid in a Murine Model. Antimicrobial Agents and Chemotherapy 53:1314-1319.
19. Alffenaar JW, van Altena R, Harmelink IM., Filguers P, Molenaar E, Wessels AM, van Soolingen D, Kosterink JG, Uges DR, van der Werf TS. 2010. Comparison of the pharmacokinetics of two dosage regimens of linezolid in multidrug-resistant and extensively drug-resistant tuberculosis patients. Clin Pharmacokinet 49:559-565.
20. McGee B, Dietze R, Hadad DJ, Molino LP, Maciel EL, Boom WH, Palaci M, Johnson JL, Peloquin CA. 2009. Population pharmacokinetics of linezolid in adults with pulmonary tuberculosis. Antimicrob Agents Chemother 53:3981-3984.
21. Keel RA, Crandon JL, Nicolau DP. 2012. Pharmacokinetics and pulmonary disposition of tedizolid and linezolid in a murine pneumonia model under variable conditions. Antimicrob Agents Chemother 56:3420-3422.
22. Keel RA, Tessier PR, Crandon JL, Nicolau DP. 2012. Comparative efficacies of human simulated exposures of tedizolid and linezolid against Staphylococcus aureus in the murine thigh infection model. Antimicrob Agents Chemother 56:4403-4407.
23. Louie A, Liu W, Kulawy R, Drusano GL. 2011. In vivo pharmacodynamics of torezolid phosphate (TR-701), a new oxazolidinone antibiotic, against methicillin-susceptible and methicillin-resistant Staphylococcus aureus strains in a mouse thigh infection model. Antimicrob Agents Chemother 55:3453-3460.
24. Reele S, Xiao AJ, Das S, Balasubramanian V, Melnick D. 2011. A 14-day multiple ascending dose study: AZD5847 is well tolerated at predicted exposure for treatment of tuberculosis (TB), A1-1735., abstr 51st Intersci Conf Antimicrob Agents Chemother, Chicago, IL.
25. Wallis RS, Diacon AH, Dawson R, Venter A, Friedrich SO, Paige D, Zhu T, Silvia A, Gobey J, Ellery C, Zhang Y, Kadyezewski E. 2012. Safety, tolerability and early bactericidal activity in sputum of PNU-100480 (sutezolid) in patients with pulmonary tuberculosis, abstr XIX International AIDS Conference, Washington, D.C.
26. Sotgiu G, Centis R, D'Ambrosio L, Alffenaar JW, Anger HA, Caminero JA, Castiglia P, De Lorenzo S, Ferrara G, Koh WJ, Schecter GF, Shim TS, Singla R, Skrahina A, Spanevello A, Udwadia ZF, Villar M, Zampogna E, Zellweger JP, Zunua A, Migliori GB. 2012. Efficacy, safety and tolerability of linezolid containing regimens in treating MDR-TB and XDR-TB: systematic review and meta-analysis. Eur Respir J 40:1430-1442.
27. Chang KC, Yew WW, Tam CM, Leung CC. 2013. WHO group 5 drugs and difficult multidrug-resistant tuberculosis: a systematic review with cohort analysis and meta-analysis. Antimicrob Agents Chemother 57:4097-4104.
28. Lee M, Lee J, Carroll MW, Choi H, Min S, Song T, Via LE, Goldfeder LC, Kang E, Jin B, Park H, Kwak H, Kim H, Jeon HS, Jeong I, Job JS, Chen RY, Olivier KN, Shaw PA, Follmann D, Song SD, Lee JK, Lee D, Kim CT, Dartois Y, Park SK, Cho SN, Barry CE, 3rd, 2012. Linezolid for treatment of chronic extensively drug-resistant tuberculosis. N Engl J Med 367:1508-1518.
29. Flanagan S, McKee EE, Das D, Tulkens PM, Hosako H, Fiedler-Kelly J, Passarell J, Radovsky A, Prokocimer P. 2015. Nonclinical and pharmacokinetic assessments to evaluate the potential of tedizolid and linezolid to affect mitochondrial function. Antimicrob Agents Chemother 59:178-185.
30. Schlosser MJ, Hosako H, Radovsky A, Butt MT, Draganov D, Vija J, Oleson F. 2015. Lack of neuropathological changes in rats administered tedizolid phosphate for nine months. Antimicrob Agents Chemother 59:475-481.
31. Converse PJ, Lee J, Williams KN, Tasneen R, Dionne K, Parish N, Wallis RS, Nuermberger E. 2012. Activity of PNU-100480 and its major metabolite in whole blood and broth culture models of tuberculosis, #1563, abstr 112th American Society for Microbiology General Meeting, San Francisco, CA.
32. Zhu T, Friedrich SO, Diacon A, Wallis RS. 2014. Population Pharmacokinetic/Pharmacodynamic Analysis of the Bactericidal Activities of Sutezolid (PNU-100480) and Its Major Metabolite against Intracellular Mycobacterium tuberculosis in Ex Vivo Whole-Blood Cultures of Patients with Pulmonary Tuberculosis. Antimicrob Agents Chemother 58:3306-3311.
33. Lanoix JP, Leαaerts AJ, Nuermberger EL. 2015. Heterogeneous disease progression and treatment response in a C3HeB/FeJ mouse model of tuberculosis. Dis Model Mech 8:603-610.
34. Tessier PR, Keel RA, Hagihara M, Crandon JL, Nicolau DP. 2012. Comparative in vivo efficacies of epithelial lining fluid exposures of tedizolid, linezolid, and vancomycin for methicillin-resistant Staphylococcus aureus in a mouse pneumonia model. Antimicrob Agents Chemother 56:2342-2346.

It is to be understood that the invention is not limited to the particular embodiments of the invention described above, as variations of the particular embodiments may be made and still fall within the scope of the appended claims.

### The present invention also relates to the following items;

1. A pharmaceutical composition, comprising a therapeutically effective amount of each of linezolid, bedaquiline and pretomanid, and optionally pyrazinamide, or a pharmaceutically acceptable salt of each thereof, and a pharmaceutically acceptable carrier.
2. The pharmaceutical composition according to item 1, wherein linezolid is at a dosage of 100 mg/kg.
3. The pharmaceutical composition according to item 1, wherein linezolid is at a dosage of 50 mg/kg.
4. A method for the treatment of tuberculosis, comprising the step of administering to a patient in need thereof a therapeutically effective amount of each of linezolid, bedaquiline and pretomanid, and optionally pyrazinamide, or a pharmaceutically acceptable salt of each thereof, and a pharmaceutically acceptable carrier.
5. The method according to item 4, wherein linezolid is administered for up to three months.
6. The method according to item 4, wherein linezolid is administered for up to two months.
7. The method according to item 4, wherein linezolid is administered for up to one month.
8. The method according to item 4, wherein linezolid is administered for up to one to two months at 100 mg/kg per day.
9. The method according to item 4, wherein linezolid is administered for up to two months at 50 mg/kg per day.
10. The method according to item 4, wherein linezolid is administered for up to one month at 100 mg/kg per day.
11. The method according to item 4, wherein linezolid is administered at a dosage of 600 mg once daily for the first one to two months.
12. The method according to item 4, wherein linezolid is re-administered after a one to two week drug holiday.
13. The pharmaceutical composition according to item 1, wherein bedaquiline is at a dosage of 200 to 400 mg qd.
14. The method according to item 4, wherein bedaquiline is administered at 400 mg qd for two weeks followed by 200 mg tiw.
15. The pharmaceutical composition according to item 1, wherein linezolid is at a dosage of 600 mg bid or 1200 mg qd.
16. The pharmaceutical composition according to item 1, wherein pretomanid is at a dosage of 100 to 200 mg qd.

## Claims

1. A pharmaceutical composition which is in a unitary dosage form, comprising a therapeutically effective amount of each of linezolid, bedaquiline and pretomanid, and optionally pyrazinamide, or a pharmaceutically acceptable salt of each thereof, and a pharmaceutically acceptable carrier, wherein the linezolid, bedaquiline and pretomanid are each present in the unitary dosage form in an amount of 1 mg to 1 g.

2. The pharmaceutical composition according to claim 1, wherein the linezolid, bedaquiline and pretomanid are each present in the unitary dosage form in an amount of 10 mg to 500 mg.

3. The pharmaceutical composition according to claim 1, wherein the linezolid, bedaquiline and pretomanid are each present in the unitary dosage form in an amount of 50 mg to 300 mg .

4. The pharmaceutical composition according to claim 1, wherein bedaquiline is present in the unitary dosage form in an amount of 200 to 400 mg.

5. The pharmaceutical composition according to claim 1, wherein linezolid is present in the unitary dosage form in an amount of 600 mg.

6. The pharmaceutical composition according to claim 1, wherein pretomanid is present in the unitary dosage form in an amount of 100 to 200 mg.

7. The pharmaceutical composition according to claim 1, wherein the unitary dosage form is a tablet or capsule.

8. The pharmaceutical composition according to claim 1, wherein the single dosage form comprises 100 mg of pretomanid.

9. The pharmaceutical composition according to claim 1, wherein the single dosage form comprises 200 mg of pretomanid.

10. The pharmaceutical composition according to claim 1, wherein the fixed dosage form comprises 600 mg of linezolid and 100 mg of pretomanid.

11. The pharmaceutical composition according to claim 1, wherein the dosage form comprises 600 mg of linezolid and 200 mg of pretomanid.

12. The pharmaceutical composition according to any one of claims 1 to 11 for use in the treatment of tuberculosis.

13. The pharmaceutical composition for use according to claim 12, wherein the composition is to be administered for up to three months.

14. The pharmaceutical composition for use according to claim 12, wherein the composition is to be administered for up to two months, preferably for up to one month.

15. The pharmaceutical composition for use according to claim 12, wherein the composition is to be re-administered after a one to two week drug holiday.
